# EUROPEAN PATENT APPLICATION

(11) **EP 2 336 369 A1**
(43) Date of publication of application: **22.06.2011**
(21) Application number: 09179261.4
(22) Date of filing: 15.12.2009
(51) Int. Cl.: C12Q 1/70

(54) **Probes for genotyping low-risk-HPV**

(71) Applicant: Deutsches Krebsforschungszentrum, 69120 Heidelberg (DE)
(72) Inventor: Schmitt, Markus, 69121 Heidelberg (DE); Pawlita, Michael, 74927 Eschelbronn (DE)
(74) Representative: Stößel, Matthias

(57) **Abstract**

The current invention is concerned with a composition comprising at least one probe oligonucleotide each for the nucleotide sequences of the invention, said probe oligonucleotides specifically hybridizing to the sense strand or the antisense strand of said nucleotide sequences. Moreover, the present invention relates to a method for the identification of low-risk HPV types in a sample comprising the steps of a) contacting a sample with an amplification composition allowing amplification of at least one region of the HPV genome specifically hybridizing to at least one of the probe oligonucleotides of the current invention under conditions which allow for the amplification of polynucleotides and b) identifying low-risk HPV genotypes in said sample based on the amplified polynucleotides obtained in step a) by hybridizing the amplified polynucleotides with at least one labelled probe oligonucleotide of the current invention while said amplified polynucleotides are present in the same reaction container. The invention also relates to the use of a composition of the current invention for identifying low-risk HPV in a sample and to a kit comprising the composition of the invention and / or adopted for carrying out the method of the invention and an instruction manual.

SEQUENCE LISTING

<110>  Deutsches Krebsforschungszentrum

<120>  Probes for genotyping low-risk-HPV after GP5+/6+ or BSGP5+/6+ PCR

<130>  DK66753EP

<160>  35

<170>  PatentIn version 3.5

<210>  1
<211>  19
<212>  DNA
<213>  HPV_6

<400>  1
tccgtaacta catcttcca                                                    19

<210>  2
<211>  20
<212>  DNA
<213>  HPV_7

<400>  2
caccatatga caatagtaag                                                   20

<210>  3
<211>  20
<212>  DNA
<213>  HPV_11

<400>  3
tctgtgtcta aatctgctac                                                   20

<210>  4
<211>  20
<212>  DNA
<213>  HPV_13

<400>  4
atcatctctt tcagacacat                                                   20

<210>  5
<211>  19
<212>  DNA
<213>  HPV_30

<400>  5
cacacaaacg ttatccaca                                                    19

<210>  6
<211>  19
<212>  DNA
<213>  HPV_32

<400>  6
aacaactgaa gacacatac                                                    19

<210>  7
<211>  20
<212>  DNA

<213>  HPV_34

<400>  7
aagtacaact gcaccatatg                                                    20


<210>  8
<211>  20
<212>  DNA
<213>  HPV_40

<400>  8
ccccatataa taacagtaat                                                    20


<210>  9
<211>  19
<212>  DNA
<213>  HPV_42

<400>  9
gccactgcaa catctggtg                                                     19


<210>  10
<211>  18
<212>  DNA
<213>  HPV_43

<400>  10
tctactgacc ctactgtg                                                      18


<210>  11
<211>  21
<212>  DNA
<213>  HPV_44

<400>  11
tactagtgaa caatataagc a                                                  21


<210>  12
<211>  19
<212>  DNA
<213>  HPV_54

<400>  12
cacgcaggat agctttaat                                                     19


<210>  13
<211>  20
<212>  DNA
<213>  HPV_55

<400>  13
tcagtctcca tctacaacat                                                    20


<210>  14
<211>  18
<212>  DNA
<213>  HPV_61

<400>  14
ccccccctgt atctgaat                                                      18

<210>  15
<211>  17
<212>  DNA
<213>  HPV_62

<400>  15
aatttttgcg acacacg                                                    17


<210>  16
<211>  20
<212>  DNA
<213>  HPV_64

<400>  16
aagtacaaat ccaccatatg                                                 20


<210>  17
<211>  19
<212>  DNA
<213>  HPV_67

<400>  17
ggaaaaatca gaggctaca                                                  19


<210>  18
<211>  19
<212>  DNA
<213>  HPV_69

<400>  18
catctgccac ttttaaacc                                                  19


<210>  19
<211>  19
<212>  DNA
<213>  HPV_70

<400>  19
tttacattgt ctgcctgca                                                  19


<210>  20
<211>  19
<212>  DNA
<213>  HPV_71

<400>  20
tgctaccaaa actgttgag                                                  19


<210>  21
<211>  19
<212>  DNA
<213>  HPV_72

<400>  21
agcgtcctct gtatcagaa                                                  19


<210>  22
<211>  19
<212>  DNA
<213>  HPV_74

<400>  22

cacaatcgcc ttctgctac                                                    19

<210>  23
<211>  19
<212>  DNA
<213>  HPV_81

<400>  23
agaatacaag gcctctaac                                                    19

<210>  24
<211>  20
<212>  DNA
<213>  HPV_83

<400>  24
tacacaggct aatgaataca                                                   20

<210>  25
<211>  19
<212>  DNA
<213>  HPV_84

<400>  25
tgctaccaac accgaatca                                                    19

<210>  26
<211>  19
<212>  DNA
<213>  HPV_85c

<400>  26
aatacacacg ccatgtaga                                                    19

<210>  27
<211>  21
<212>  DNA
<213>  HPV_86c

<400>  27
tacccagaag gcctctgaat a                                                 21

<210>  28
<211>  20
<212>  DNA
<213>  HPV_87c

<400>  28
ctgccactca aacaaccact                                                   20

<210>  29
<211>  20
<212>  DNA
<213>  HPV_89/CP6108c

<400>  29
acagaataca gttctacacg                                                   20

<210>  30
<211>  19
<212>  DNA

<213>  HPV_90

<400>  30
aacaccctct gacacatac                                                    19


<210>  31
<211>  20
<212>  DNA
<213>  HPV_91

<400>  31
catccactga gtctgtgcta                                                   20


<210>  32
<211>  19
<212>  DNA
<213>  HPV_97

<400>  32
caaaatggcg tagctacca                                                    19


<210>  33
<211>  19
<212>  DNA
<213>  HPV_102

<400>  33
tcacagtccg gtgaatacc                                                    19


<210>  34
<211>  20
<212>  DNA
<213>  HPV_106

<400>  34
acgcaaacag aaaatgccac                                                   20


<210>  35
<211>  19
<212>  DNA
<213>  HPV_114

<400>  35
cctccgctac ccaggcctc                                                    19

## Description

The current invention is concerned with a composition comprising at least one probe oligonucleotide each for the nucleotide sequences of the invention, said probe oligonucleotides specifically hybridizing to the sense strand or the antisense strand of said nucleotide sequences. Moreover, the present invention relates to a method for the identification of low-risk HPV types in a sample comprising the steps of a) contacting a sample with an amplification composition allowing amplification of at least one region of the HPV genome specifically hybridizing to at least one of the probe oligonucleotides of the current invention under conditions which allow for the amplification of polynucleotides and b) identifying low-risk HPV genotypes in said sample based on the amplified polynucleotides obtained in step a) by hybridizing the amplified polynucleotides with at least one labelled probe oligonucleotide of the current invention while said amplified polynucleotides are present in the same reaction container. The invention also relates to the use of a composition of the current invention for identifying low-risk HPV in a sample and to a kit comprising the composition of the invention and / or adopted for carrying out the method of the invention and an instruction manual.

Human papillomaviruses form a large group of viruses and are small, non-enveloped DNA viruses that infect almost exclusively skin and mucosal cells. To date, the genome of almost 100 genotypes of human papillomaviruses has been characterized (de Villiers, E. M., C. Fauquet, T. R. Broker, H. U. Bernard, and H. zur Hausen (2004). Classification of papillomaviruses. Virology 324:17-27), and a much larger number of human papillomaviruses is thought to exist (Hazard K, Andersson K, Dillner J, Forslund O. (2007). Human papillomavirus subtypes are not uncommon. Virology. May 25;362(1):6-9). Approximately 50 HPV genotypes are known to infect the mucosa. These mucosal genotypes are classified into three different groups based on their epidemiological association with cancer: "low-risk" human papillomaviruses (LR-HPV), "high-risk" human papillomaviruses (HR-HPV) and "putative high-risk" human papillomaviruses (pHR-HPV). Low-risk human papillomaviruses (including, e.g., HPV genotypes 6, 11, 40, 42, 43, 44 and 70) are primarily found in genital warts and low-grade cervical lesions. While it has been estimated that a minimum of approximately 13,000 clinical lesions per year are associated with HPV-6 and HPV-11, two LR-HPV types, in Finland alone (Syrjänen KJ (2009). Annual disease burden due to human papillomavirus (HPV) 6 and 11 infections in Finland. Scand J Infect Dis Suppl. 107: 3-32), a causative relationship of low-risk HPV types with tumors is still controversial.

In order to verify or falsify such a causative relationship, the ability to identify specific HPV genotypes present in a sample is important. Moreover, the ability to determine the specific type of HPV present in a clinical sample is of value for the gynecologist, as various HPV genotypes may pose different risks to the affected patients. To this end, several detection systems have been devised that typically rely on the detection of HPV-related nucleic acids. Among these, PCR-based methods were developed allowing a more precise detection of HPV infection. The majority of these PCR systems use consensus or general primers that bind to highly conserved regions of the HPV genome. WO 95/22626 describes the use of two primers, GP5+ and GP6+, for the detection and identification of different HPV genotypes. In comparison to GP5 and GP6 (WO 91/10675), GP5+ and GP6+ primers possess elongated 3' ends improving human papillomavirus detection by PCR (Jacobs, M. V., J. M. Walboomers, P. J. Snijders, F. J. Voorhorst, R. H Verheijen, N. Fransen-Daalmeijer, and C. J. Meijer. 2000. Distribution of 37 mucosotropic HPV types in women with cytologically normal cervical smears: the age-related patterns for high-risk and low-risk types. Int J Cancer 87:221-7). EP 2034032 describes an improved set of primers (BSGP5+ / BSGP6+) which allows a more representative amplification in the case that more than one HPV genotype is present in a sample. All of the aforementioned primers are directed to conserved sequence regions within the L1 region of the HPV genome.

The amplified PCR products are then subjected to further analysis (e.g. sequencing, restriction fragment length polymorphism (RFLP) analysis or hybridization) in order to identify specific low-risk mucosal HPV genotypes. However, to date, only a fraction of the known low-risk HPV types can be identified with the tests available.

Thus, the technical problem underlying the present invention may be seen as the provision of means and methods for efficiently and reliably detecting and diagnosing different low-risk human papillomavirus (HPV) genotypes without the drawbacks as referred to above. The technical problem is solved by the embodiments characterized in the claims and herein below. Accordingly, the current invention relates to a composition comprising at least one probe oligonucleotide each for the nucleotide sequences of SEQ ID NOs: 1 to 35, said probe oligonucleotides specifically hybridizing to the sense strand or the antisense strand of said nucleotide sequences.

The term "composition" as meant herein relates to a mixture of different oligonucleotide molecular species. Preferably, the mixture in addition comprises further components other than the oligonucleotides, e.g. solvents (including water, Dimethylsulfoxide, Dimethylformamide, Ethanol, or Methanol), salts, detergents, or components comprised in the amplification mixture used for the amplification of polynucleotides of mucosal HPV genotypes, preferably by polymerase chain reaction (PCR). Such components may be, but are not limited to, an aqueous buffer, a water soluble magnesium salt, deoxythymidine triphosphate (dTTP), deoxyadenosine triphosphate (dATP), deoxycitidine triphosphate (dCTP) and deoxyguanosine triphosphate, (dGTP) and a DNA polymerase, e.g. the thermostable DNA polymerase from Thermus aquaticus It is, however, also contemplated by the current invention that the oligonucleotide molecular species of the composition are separated, e.g. separated spatially in an array that allows the identification of each oligonucleotide molecular species by means of determining the relevant position on the array, like e.g. in a microarray or on a line or dot blot.

As used herein, the term "sense strand of one of the nucleotide sequences of SEQ ID NOs: 1 to 35" relates to an oligonucleotide identical in sequence with one of the nucleotide sequences of SEQ ID NOs: 1 to 35; the term "antisense strand of one of the nucleotide sequences of SEQ ID NOs: 1 to 35" relates to an oligonucleotide complementary in sequence to one of the nucleotide sequences of SEQ ID NOs: 1 to 35.

A "probe oligonucleotide" in the context of the present invention, preferably, is a single-stranded nucleic acid molecule that specifically hybridizes with the sense strand or the antisense strand of one of the nucleotide sequences of SEQ ID NOs: 1 to 35. Thus, a probe oligonucleotide comprises a stretch of nucleotides that specifically hybridize with the sense strand or the antisense strand of one of the nucleotide sequences of SEQ ID NOs: 1 to 35. Said stretch of nucleotides is, preferably, 85%, 90%, 95%, 99% or, more preferably, 100% identical to the sense strand or the antisense strand of one of the nucleotide sequences of SEQ ID NOs: 1 to 35. The percent identity values are, preferably, calculated over the entire nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit [Needleman and Wunsch (J. Mol. Biol. 48; 443-453 (1970)) and Smith and Waterman (Adv. Appl. Math. 2; 482-489 (1981))], which are part of the GCG software packet [Genetics Computer Group, 575 Science Drive, Madison, Wisconsin, USA 53711 (1991)], are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

Preferably, a specific probe oligonucleotide is a specific indicator for the HPV genotype associated with the nucleotide sequence. Preferably, a probe oligonucleotide as meant herein is between 10 and 50 nucleotides in length, more preferably said probe oligonucleotide is between 15 and 30 nucleotides in length, and most preferably between 18 and 23 nucleotides in length. Preferably, the probe oligonucleotides are bound to a carrier providing a solid surface. More preferably, said carrier is a small particle or bead. The overall size of a small particle or bead, preferably, may be in the micrometer or nanometer range. Preferably, said beads and particles may be stained with a specific dye, more preferably with a specific fluorescent dye. Preferably, by staining various carriers with various dyes, the carriers can be distinguished from each other. By using a carrier with a specific dye for a specific probe oligonucleotide (thus, a nucleic acid that targets the amplified polynucleotides of a specific low-risk HPV type), said carrier is distinguishable from other carriers comprising different dyes. In one preferred embodiment, commercially available Luminex microspheres (Luminex Corp., Austin, Texas, USA) are used. Thus, for determining LR- HPV types, the LR- HPV type-specific probes are coupled to fluorescence-labelled polystyrene beads (Luminex suspension array technology) which are hybridized with amplification products as specified herein below under suitable, preferably, stringent conditions. It is, however, also contemplated by the current invention that the probe oligonucleotides are linked to a suitable carrier in a spatially separated way, e.g. in the form of microarrays, Reverse-Line blots (RLB), dot blots or similar technologies.

Preferably, the probe oligonucleotides are selected from the group consisting of probe oligonucleotides comprising a nucleic acid sequence as shown in SEQ ID NO:1 (allows the detection of HPV 6), SEQ ID NO:2 (allows the detection of HPV 7), SEQ ID NO:3 (allows the detection of HPV 11), SEQ ID NO:4 (allows the detection of HPV 13), SEQ ID NO:5 (allows the detection of HPV 30), SEQ ID NO:6 (allows the detection of HPV 32, and SEQ ID NO:7 (allows the detection of HPV 34), SEQ ID NO:8 (allows the detection of HPV 40), SEQ ID NO:9 (allows the detection of HPV 42), SEQ ID NO:10 (allows the detection of HPV 43), SEQ ID NO:11 (allow the detection of HPV 44), SEQ ID NO: 12 (allows the detection of HPV 54), SEQ ID NO:13 (allows the detection of HPV 55, SEQ ID NO:14 (allows the detection of HPV 61), SEQ ID NO:15 (allows the detection of HPV 62), SEQ ID NO:16 (allows the detection of HPV 64), SEQ ID NO: 17 (allows the detection of HPV 67), SEQ ID NO: 18 (allows the detection of HPV 69), SEQ ID NO:19 (allows the detection of HPV 70), SEQ ID NO:20 (allows the detection of HPV 71), SEQ ID NO:21 (allows the detection of HPV 72), SEQ ID NO:22 (allows the detection of HPV 74), SEQ ID NO:23 (allows the detection of HPV 81), SEQ ID NO:24 (allows the detection of HPV 83), SEQ ID NO:25 (allows the detection of HPV 84), SEQ ID NO:26 (allows the detection of HPV 85c), SEQ ID NO:27 (allows the detection of HPV 86c), SEQ ID NO:28 (allows the detection of HPV 87c), SEQ ID NO:29 (allows the detection of HPV 89/CP6108c), SEQ ID NO:30 (allows the detection of HPV 90), SEQ ID NO:31 (allows the detection of HPV 91), SEQ ID NO:32 (allows the detection of HPV 97), SEQ ID NO:33 (allows the detection of HPV 102), and SEQ ID NO:34 (allows the detection of HPV 106), and SEQ ID NO:35 (allows the detection of HPV 114).

Preferably, the composition of the current invention comprises at least one probe oligonucleotide each for the nucleotide sequences of SEQ ID NOs: 1 to 35. More preferably, said composition comprises one probe oligonucleotide each for the nucleotide sequences of SEQ ID NOs: 1 to 35. It is, however, also contemplated that the composition of the current invention lacks for one, two, three, four, five, six, seven, eight, nine, ten, eleven, twelve, thirteen, fourteen, or fifteen probe oligonucleotide(s) specifically hybridizing to the sense strand or the antisense strand of SEQ ID NOs: 1 to 35, i.e. said number of sequences selected from SEQ ID NOs: 1 to 35 may not be represented in the composition of the current invention.

The probe oligonucleotides of the present invention may be labelled or contain other modifications which allow a detection and/or analysis of a hybridization product and/or the binding to a carrier. Labelling can be done by various techniques well known in the art and depending of the label to be used. Particularly, the probe oligonucleotides may be biotinylated in order to enable binding to a streptavidin surface or fluorescent conjugate. Exemplary labels to be used in the context of the present invention are, but are not limited to, fluorescent labels comprising, inter alia, fluorochromes such as R-phycoerythrin, Cy3, Cy5, fluorescein, rhodamin, Alexa, or Texas Red. However, the label may also be an enzyme or an antibody. It is envisaged that an enzyme to be used as a label will generate a detectable signal by reacting with a substrate. Suitable enzymes, substrates and techniques are well known in the art. An antibody to be used as label may specifically recognize a target molecule which can be detected directly (e.g., a target molecule which is itself fluorescent) or indirectly (e.g., a target molecule which generates a detectable signal, such as an enzyme). The probe oligonucleotides of the present invention may also contain 5' restriction sites, locked nucleic acid molecules (LNA) or be part of a peptide nucleotide acid molecule (PNA). Such PNA can be, in principle, detected via the peptide part by, e. g., antibodies.

As used herein, the term "individual labels" relates to labels that, preferably, allow probe oligonucleotide molecular species to be distinguished from each other. More preferably, at least two, at least three, at least four, at least five, at least six, at least seven, at least eight, at least nine, or at least ten probe oligonucleotide molecular species can be distinguished with individual labels. More preferably, individual labelling is used in combination with spatial separation to allow specific detection of all probe oligonucleotide molecular species present in the composition of the current invention. Most preferably, specific oligonucleotide molecular species are coupled to specifically fluorescence-labelled polystyrene beads (Luminex beads) as described herein above.

In another preferred embodiment, the current invention relates to a method for the identification of LR-HPV types in a sample comprising the steps of a) contacting a sample with an amplification composition allowing amplification of at least one region of the HPV genome specifically hybridizing to at least one of the probe oligonucleotides of any one of claims 1 to 5 under conditions which allow for the amplification of polynucleotides and b) identifying low-risk HPV genotypes in said sample based on the amplified polynucleotides obtained in step a) by hybridizing the amplified polynucleotides with at least one labelled probe oligonucleotide of claim 4 or 5 while said amplified polynucleotides are present in the same reaction container.

As used herein, the term "low-risk human papillomavirus (LR-HPV)" relates to types of human papillomaviruses infecting mucosa, for which an increased risk of carcinogenesis correlated with infection has not been established by scientific means. Preferably, LR-HPV types are HPV 6, 7, 11, 13, 30, 32, 34, 40, 42, 43, 44, 54, 55, 61, 62, 64, 67, 69, 70, 71, 72, 74, 81, 83, 84, 85, 86, 87, 89 (CP6108), 90, 91, 97, 102, 106, and 114.

The term "contacting" as used in the context of the methods of the present invention is understood by the skilled person. Preferably, the term relates to bringing a composition of the present invention in physical contact with a sample and thereby, e.g. allowing the sample and the composition to interact. Contacting may further involve lysing cells present in the sample and/or the extraction or purification of DNA or RNA.

The term "sample" refers to a sample of a body fluid, to a sample of separated cells or to a sample from a tissue or an organ or to a sample of wash/rinse fluid obtained from an outer or inner body surface. The samples comprises polynucleotides, preferably the sample comprises DNA. Samples can be obtained by well known techniques and include, preferably, scrapes, swabs or biopsies from the urogenital tract, perianal regions, anal canal, the oral cavity, the upper aerodigestive tract and the epidermis. Such samples can be obtained by use of brushes, (cotton) swabs, spatula, rinse/wash fluids, punch biopsy devices, puncture of cavities with needles or surgical instrumentation. Preferably, the scrapes contain mucosal cells. However, samples of blood, plasma, serum, urine, saliva, lacrimal fluid, stool are also encompassed by the method of the present invention. Tissue or organ samples may be obtained from any tissue or organ by, e.g., biopsy or other surgical procedures. Separated cells may be obtained from the body fluids or the tissues or organs by separating techniques such as filtration, centrifugation or cell sorting. Preferably, cell, tissue or organ samples are obtained from those cells, tissues or organs which are known or suspected targets of alpha genus HPV genotypes, more preferably mucosal HPV genotypes, and, therefore, may comprise HPV-specific polynucleotides. It is to be understood that the sample may be further processed in order to carry out the method of the present invention. Particularly, polynucleotides such as DNA or RNA, preferably DNA, might be extracted and/or purified from the obtained sample by methods and means known in the art. Thus, the term sample also may relate to polynucleotides, preferably DNA, purified and/or extracted from any sample as mentioned to above.

As used herein, the term "amplification composition" refers to a composition comprising components required to amplify the polynucleic acids comprised in a sample. Preferably, polynucleic acids are amplified by PCR and the amplification composition comprises, e.g. an aqueous buffer, a water soluble magnesium salt, deoxythymidine triphosphate (dTTP), deoxyadenosine triphosphate (dATP), deoxycitidine triphosphate (dCTP) and deoxyguanosine triphosphate, (dGTP) and a thermostable DNA polymerase, e.g. the DNA polymerase from Thermus aquaticus.

The term "region of the HPV genome specifically hybridizing to at least one of the probe oligonucleotides" relates to a region on one of the HPV genomes of the current invention with the capacity to specifically hybridize to an oligonucleotide having the sequence of one of SEQ ID NOs: 1 to 35, or to the antisense strand of one of SEQ ID NOs: 1 to 35. Preferably, said region is the region amplified by the MY09 / MY 11, the PGMY09 / PGMY 11, theGP5 / GP6 or the GP5+ / GP6+ primers; more preferably, said region is the region amplified by the BSGP5+ / BSGP6+ primers.

The term "conditions which allow for the amplification of polynucleotides" relates to conditions that allow the amount of polynucleotides to increase. Preferably, conditions are selected to cause the amount of polynucleotides comprising at least one region of the HPV genome specifically hybridizing to at least one of the probe oligonucleotides to increase compared to the total amount of polynucleotides. More preferably, the increase is specific for polynucleic acids amplified by the MY09 / MY 11, the PGMY09 / PGMY 11, the GP5 / GP6 or the GP5+ / GP6+ primers or their combinations. Most preferably, the increase is specific for polynucleic acids amplified by the BSGP5+ / BSGP6+ primers.

The term "identifying low-risk HPV genotypes based on the amplified polynucleotides" relates to identifying amplified polynucleotides that are specific for the various, individual HPV genotypes as specified elsewhere in this application. Preferably, this will be achieved by detecting the presence or absence of amplified polynucleotides being specific for the HPV genotypes to be detected. If an amplified polynucleotide that is specific for an individual HPV genotype is present, then the presence of an infection with the respective HPV genotype can be diagnosed. If there is no amplification product for a polynucleotide that is specific for a certain HPV genotype (thus, if an amplified polynucleotide is absent), then the absence of an infection with the respective HPV genotype can be diagnosed.

Identifying the different HPV genotypes based on the amplified polynucleotides, preferably, is based on genotype specific sequence variations in the amplified polynucleotides. Preferably, the presence or absence of amplified polynucleotides and, therefore, of different HPV genotypes is assessed by methods involving hybridization to poly- or oligonucleotides that are complementary to individual genotypic sequences in the amplified polynucleotides. Such poly- and oligonucleotides are described in the Examples. The hybridization and the detection of the occurrence of a hybridization event may be carried out by any method under any conditions deemed appropriate, e.g., by Southern blot assays, dot blot assays, or by membrane-based reverse line blot (Melchers et al., Prevalence of genital HPV infections in a regularly screened population in The Netherlands in relation to cervical cytology. 1988. J Med Virol 25:11-6; van den Brule et al., GP5+/6+ PCR followed by reverse line blot analysis enables rapid and high-throughput identification of human papillomavirus genotypes. 2002. J Clin Microbiol 40:779-87; Melchers et al., Optimization of human papillomavirus genotype detection in cervical scrapes by a modified filter in situ hybridization test. 1989. J Clin Microbiol 27:106-10). A particularly preferred method for the detection and identification of HPV genotype specific amplification products is a modification of the Multiplex HPV Genotyping (MPG) assay: the probe oligonucleotides of the current specification are coupled to fluorescence-labelled polystyrene beads (Luminex suspension array technology) which are hybridized with the amplification products under suitable, preferably, stringent conditions. Moreover, the amplification products may be identified by use of DNA-Chips which contain HPV probe oligonucleotides linked to a suitable carrier.

As used herein "reaction container" relates to a container comprising the amplified polynucleotides and the probe oligonucleotides of the present invention. Preferably, the reaction container is crafted in a way to allow its contents to be kept under conditions allowing specific hybridization. E.g. the conatiner may be a reaction tube or a well in a multiwell plate. In other embodiments the container can, e.g. be a tray accomodating at least one blotting membrane or at least one DNA-chip.

In another preferred embodiment, the current invention relates to the use of a composition of the current invention for identifying low-risk HPV in a sample. Preferably, the HPV genotypes are identified in the same reeaction container.

In a further preferred embodiment, the current invention relates to a kit comprising the composition of any one of claims 1 to 5 and / or adopted for carrying out the method of claim 6 or 7 and an instruction manual.

The term "kit" as used herein refers to a collection of the aforementioned means, e.g., a composition comprising the probe oligonucleotides of the current invention and / or means for contacting a sample under conditions which allow for amplification of polynucleotides and for determining different HPV genotypes based on the amplified polynucleotides, preferably, provided separately or within a single container. The container, also preferably, comprises instructions for carrying out the method of the present invention. The components of the kit are provided, preferably, in a "ready-to-use" manner, e.g., concentrations are adjusted accordingly, etc.

All references cited in this specification are herewith incorporated by reference with respect to their entire disclosure content and the disclosure content specifically mentioned in this specification.

The following Examples shall merely illustrate the invention. They shall not be construed, whatsoever, to limit the scope of the invention.

### Figure Legends:

Fig. 1. HPV type distribution in the Mongolian population
Fig. 2. HPV type distribution in the Mongolian SCC cases

### Examples

### Example 1.DNA isolation from cervical scrapings.

For DNA isolation, 2.0 ml (from a total of 20 ml) of cervical scrapings collected with a cytobrush (Cervex-Brush, Rovers Medical Devices B.V., 5347 KV, The Netherlands) in PreserveCytTM solution (Cytyc Corp., Boxborough, MA, USA) were purified by the Roche High Pure PCR Template Preparation Kit according to the manufacturers' instructions. DNA was eluted in 0.2 ml of elution buffer (10 mM Tris, pH 8.5) and stored at -20°C until further use. Scrapes were kept 2 months at 4°C before DNA extraction.

### Example 2.Amplification of cloned HPV DNA by GP5+/6+ and BSGP5+/6+ PCR

GP5+/6+ PCR was performed as previously described in WO 95/22626 and de Roda Husman, A. M., J. M. Walboomers, A. J. van den Brule, C. J. Meijer, and P. J. Snijders. 1995. The use of general primers GP5 and GP6 elongated at their 3' ends with adjacent highly conserved sequences improves human papillomavirus detection by PCR. J Gen Virol 76 (Pt 4):1057-62 with some modifications. Briefly, 10 µL of DNA extracted from cervical scrapings or 1 µL of HPV plasmid dilution were amplified in 50 µL containing 50 mM KCl, 0.8 g/L NonidetTM P40, 10 mM Tris HCl, pH 8.8 (10x PCR buffer, MBI Fermentas GmbH, St. Leon Roth, Germany), 200 µM of each deoxynucleoside triphosphate (dNTP), 3.5 mM MgCl₂ (Biozym Scientific GmbH, Hessisch Oldendorf, Germany), 1 U of DNA AmpliTaq polymerase (Roche Applied Biosystems, Mannheim, Germany) and 500 nM each of the GP5+ and 5'-biotinylated GP6+ primers (MWG-Biotech AG, Ebersberg, Germany). In case of the integrated b-globin/GP5+/6+ PCR, 100 nM each of the novel b-globin primers MS3fw and 5'biotinylated MS3bw were added per PCR mixture. A 4 min denaturation step at 94 °C was followed by 40 cycles of amplification with a PCR thermocycler (Gene Amp PCR System 2400, Perkin-Elmer, Wellesley, MA) or a Mastercycler (Eppendorf, Germany). Each cycle included a denaturation step at 94 °C for 20 s, an annealing step at 38 °C for 30 s, and an elongation step at 71 °C for 80 s. The final elongation step was prolonged for further 4 min. Ramping rates for the Mastercycler were adjusted as described recently (Snijders, P. J., A. J. van den Brule, M. V. Jacobs, R. P. Pol, and C. J. Meijer. 2005. HPV DNA detection and typing in cervical scrapes. Methods Mol Med 119:101-14): 1.8 °C/s from 94 °C to 38 °C, 2.0 °C/s from 38 °C to 71 °C and 2,8 °C/s from 71 °C to 94 °C. Each PCR experiment was performed with positive and several negative PCR controls.

For the broad spectrum BSGP5+/6+ PCR assay, 8 additional forward and 2 additional 5'-biotinylated backward primers were added to the GP5+/6+ PCR reaction as previously described in EP 2034032. Briefly, 200 nM of each forward (excluding GP5+), 400 nM of each backward (excluding GP6+), 500 nM of the GP5+ and 600 nM of the GP6+, and 300 nM each of the b-globin primers MS3fw and 5'-biotinylated MS3bw were used. Otherwise, the PCR buffers, reagents and amplification profiles were identical to those described above. Analytic sensitivity of the BSGP5+/6+ primers, was determined for plasmid clones of HPV 6, 11, 42, 43, 44, and 70. Plasmid preparations were quantified using NanoDrop® ND-1000 (NanoDrop Technologies, Wilmington, Delaware, USA) or a Hitachi U-1100 spectrophotometer (Hitachi, Ltd., Tokyo, Japan). The copy numbers for each HPV type were determined on the basis of the molecular weight of each of the plasmids. 10-fold endpoint dilution series were prepared in 100 ng/µL of Human placenta (HP-) DNA in a total volume of 30 µL. Two to three replicates of each dilution were assayed independently. In all experiments, 10 to 100 copies of all HPV types analysed could be detected.

### Example 3. Coupling of oligonucleotide probes.

The sequences of 5' Amino-Modifier C-12 linked oligonucleotide probes (MWG-Biotech) are shown in Table 1. Probes were coupled to carboxylated beads by a carbodiimide-based coupling procedure. For each combination of probe and bead set, 2.5 million carboxylated beads (xMAP, Luminex Corp., Austin, TX) were suspended in 25 µL of 0.1 M 2-(N-Morpholino) ethanesulfonic acid, pH 4.5 (MES). Probe oligonucleotides (400 pmol) and 200 µg of N-(3-dimethylaminopropyl)-N-ethylcarbodiimide (EDC) were added and thoroughly mixed with the beads. Incubation was performed in the dark under agitation for 30 min and was interrupted by a thorough mixing step after 15 min. The addition of EDC and incubation steps were repeated and the coupled beads were finally washed once with 0.5 mL of 0.2 g/L Tween-20 and once with 0.5 mL of 1.0 g/L SDS before being stored in 100 µL of TE buffer at 4 °C in the dark. Coupling efficiency of new bead batches compared to old ones was verified by hybridisation to 1.0 to 4.0 µL of biotinylated PCR products of the respective HPV.

### Example 4. Multiplex HPV Genotyping (MPG).

Following multiplex PCR amplification, 10 µl of each reaction mixture were transferred to 96-well plates containing in each well 33 µl of tetramethylammonium chloride (TMAC) hybridization solution (0.15 M TMAC, 75 mM Tris-HCl, 6 mM EDTA, 1.5 g/liter Sarkosyl, pH 8.0), 7.0 µl of 1 x TE, and a mixture of 2,000 probe-coupled beads of each set. The mixture was heated to 95°C for 10 min, immediately placed on ice for 1 min, and then moved to a thermomixer for hybridization at 41°C for 30 min under agitation. The samples were transferred to a 96-well wash plate (Millipore, Bedford, MA), pre-equilibrated with washing buffer (PBS, 0.02% Tween). Subsequently, the beads were washed once with 100 µl of washing buffer on a vacuum wash station (Millipore Bedford, MA). On a horizontal shaker at room temperature, beads were resuspended for 20 min in 50 µl of streptavidin-R-phycoerythrin (Molecular Probes, Eugene, OR) diluted 1:1,600 in 2.0 M TMAC, 75 mM Tris-HCl, 6 mM EDTA, 1.5 g/liter Sarkosyl, pH 8.0. Beads were then washed three times with 100 µl washing buffer and finally resuspended in 100 µl washing buffer for 5 min on a shaker. Beads were analyzed for internal bead color and R-phycoerythrin reporter fluorescence on a Luminex 100 analyzer. The median reporter fluorescence intensity (MFI) of at least 100 beads was computed for each bead set in the sample.

For each probe, MFI values in reactions with no PCR product added to the hybridisation mixture were considered background values. Net MFI values were computed by subtraction of 1.1 times the median background value. For all probes, this cut-off value was above the mean background plus three times the standard deviation. Net MFI values above 5 MFI were defined as positive reactions.

### Example 5. Specificity of the 35 low-risk HPV probes using Luminex technology

In order to determine the specificity of the 35 low-risk probes (LR- HPV types: 6, 7, 11, 13, 30, 32, 34, 40, 42, 43, 44, 54, 55, 61, 62, 64, 67, 69, 70, 71, 72, 74, 81, 83, 84, 85, 86, 87, 89(CP6108c), 90, 91, 97, 102, 106 and 114) as well as universal, and beta globin probes, all probes were designed with a 5'amino C12 linker (Table 1) and coupled individually to defined bead sets. The bead mixture was hybridised to 5 µL (∼100-300 ng DNA) of PCR products derived from bacterial colonies transformed with cloned HPV plasmid templates as described (Schmitt et al., 2008).

**Table 1. Sequences of low-risk HPV probes**

| **SEQ ID NO**: | **HPV type** | **Sequence 5'-3'** |
|---|---|---|
| 1 | 6 | TCCGTAACTACATCTTCCA |
| 2 | 7 | CACCATATGACAATAGTAAG |
| 3 | 11 | TCTGTGTCTAAATCTGCTAC |
| 4 | 13 | ATCATCTCTTTCAGACACAT |
| 5 | 30 | CACACAAACGTTATCCACA |
| 6 | 32 | AACAACTGAAGACACATAC |
| 7 | 34 | AAGTACAACTGCACCATATG |
| 8 | 40 | CCCCATATAATAACAGTAAT |
| 9 | 42 | GCCACTGCAACATCTGGTG |
| 10 | 43 | TCTACTGACCCTACTGTG |
| 11 | 44 | TACTAGTGAACAATATAAGCA |
| 12 | 54 | CACGCAGGATAGCTTTAAT |
| 13 | 55 | TCAGTCTCCATCTACAACAT |
| 14 | 61 | CCCCCCCTGTATCTGAAT |
| 15 | 62 | AATTTTTGCGACACACG |
| 16 | 64 | AAGTACAAATCCACCATATG |
| 17 | 67 | GGAAAAATCAGAGGCTACA |
| 18 | 69 | CATCTGCCACTTTTAAACC |
| 19 | 70 | TTTACATTGTCTGCCTGCA |
| 20 | 71 | TGCTACCAAAACTGTTGAG |
| 21 | 72 | AGCGTCCTCTGTATCAGAA |
| 22 | 74 | CACAATCGCCTTCTGCTAC |
| 23 | 81 | AGAATACAAGGCCTCTAAC |
| 24 | 83 | TACACAGGCTAATGAATACA |
| 25 | 84 | TGCTACCAACACCGAATCA |
| 26 | 85c | AATACACACGCCATGTAGA |
| 27 | 86c | TACCCAGAAGGCCTCTGAATA |
| 28 | 87c | CTGCCACTCAAACAACCACT |
| 29 | 89/CP6108c | ACAGAATACAGTTCTACACG |
| 30 | 90 | AACACCCTCTGACACATAC |
| 31 | 91 | CATCCACTGAGTCTGTGCTA |
| 32 | 97 | CAAAATGGCGTAGCTACCA |
| 33 | 102 | TCACAGTCCGGTGAATACC |
| 34 | 106 | ACGCAAACAGAAAATGCCAC |
| 35 | 114 | CCTCCGCTACCCAGGCCTC |

Typing of all HPV types was highly specific (Table 2). Of the 35 new low-risk probes, 33 reacted with absolute specificity. 2 probes (e.g. HPV106) showed very weak reactivity (mostly below cutoff, even when using large amounts of PCR products) with one additional HPV type each (Table 2). Using suitable algorithms any potential cross-reactivities can be easily excluded during the analysis of clinical samples. None of the HPV probes cross-reacted with the beta-globin PCR products.

Taken together, DNA detection of all HPV types examined was highly specific. The table also demonstrates the simple read-out of results due to quantitative signal values and the stability of the background, observed with all probes.

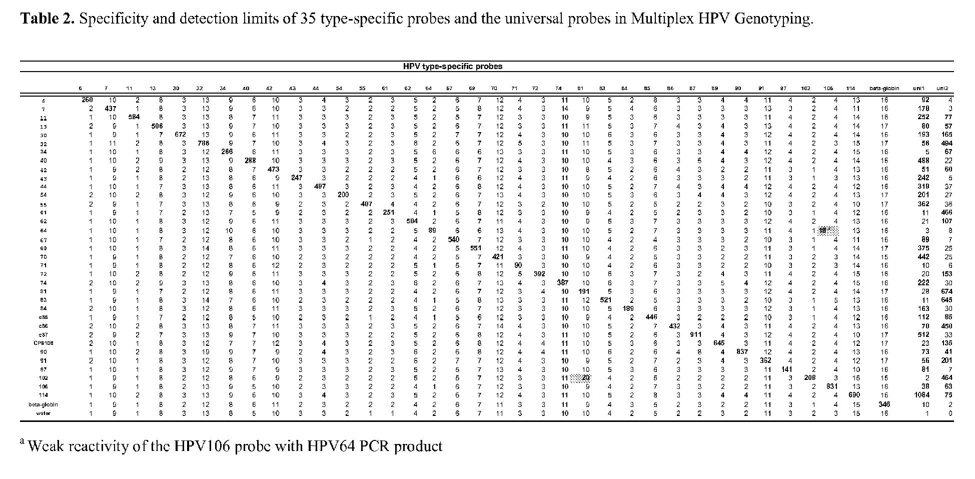

### Example 6. Prevalence of 35 low-risk HPV types in the Mongolian population and cancer series

We performed BSGP5+/6+ PCR plus integrated β-globin PCR on available DNA originating from cervical exfoliated cells from 983 women of the general population in Mongolia (Dondog et al., 2008) and 82 Mongolian cervical cancer patients. PCR Products were hybridised to all bead coupled probes described in Example 1.

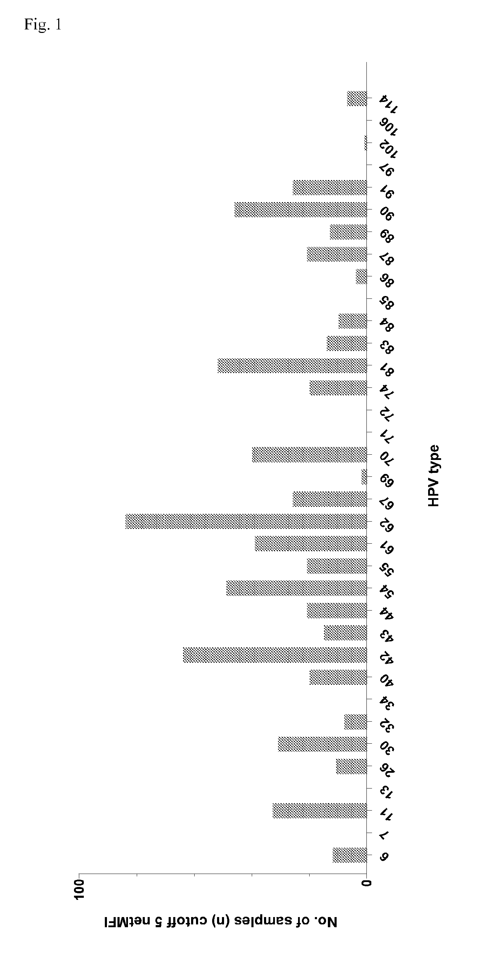

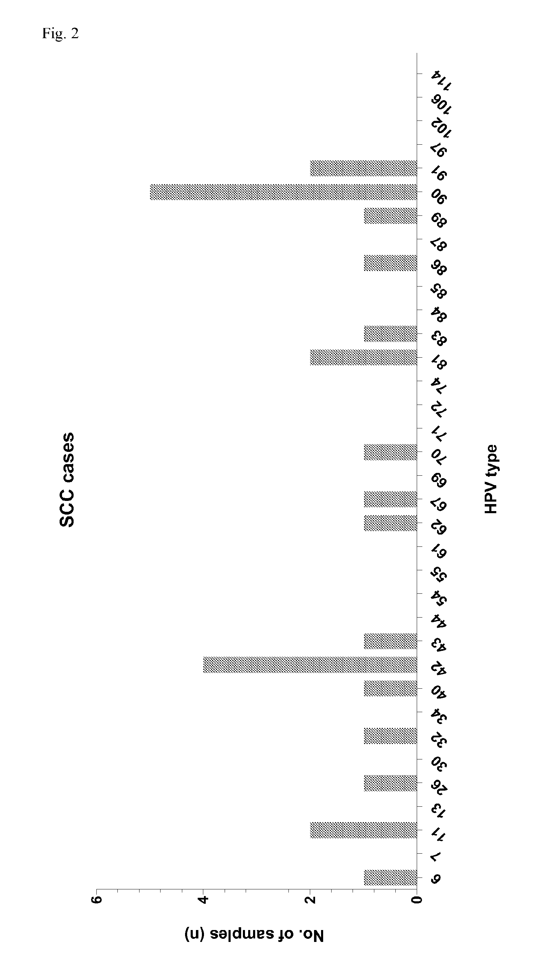

Among the population samples (Fig. 1), all low-risk HPV types were detected with varying frequency except types 7, 13, 34, 71, 72, 85 and 106 that were not found in this population. Among the 82 SCC cases, several low-risk types were frequently found with HPV90 being the most prevalent one followed by type 42 (Fig. 2). HPV70 was also detected in one single infection (data not shown). This data indicates that also low-risk HPV types may be involved in cervical cancer development and should be studied in more detail in future studies.

## Claims

1. A composition comprising at least one probe oligonucleotide each for the nucleotide sequences of SEQ ID NOs: 1 to 35, said probe oligonucleotides specifically hybridizing to the sense strand or the antisense strand of said nucleotide sequences.

2. The composition of claim 1, wherein the probe oligonucleotides have the nucleotide sequences of SEQ ID NOs: 1 to 35.

3. The composition of claim 1 or 2, wherein the composition lacks for at least one probe oligonucleotide specifically hybridizing to the sense strand or the antisense strand of SEQ ID NOs: 1 to 35.

4. The composistion of any one of claims 1 to 3, wherein the composition lacks for the probe oligonucleotides specifically hybridizing to the sense strand or the antisense strand of SEQ ID NOs: 1, 3, 5, 9, 10, 11, 17, 18, and 19.

5. The composition of any one of claims 1 to 4, wherein the probe oligonucleotides carry individual labels.

6. The composition of claim 5, wherein the label is omitted from at least one probe oligonucleotide.

7. A method for the identification of low-risk HPV types in a sample comprising the steps of
a) contacting a sample with an amplification composition allowing amplification of at least one region of the HPV genome specifically hybridizing to at least one of the probe oligonucleotides of any one of claims 1 to 6 under conditions which allow for the amplification of polynucleotides and
b) identifying low-risk HPV genotypes in said sample based on the amplified polynucleotides obtained in step a) by hybridizing the amplified polynucleotides with at least one labelled probe oligonucleotide of claim 5 or 6 while said amplified polynucleotides are present in the same reaction container.

8. The method of claim 7, wherein the amplified polynucleotides are obtained by polymerase chain reaction.

9. Use of a composition of any one of claims 1 to 6 for identifying low-risk HPV in a sample.

10. The use of claim 9, wherein different low-risk HPVs are identified in the same reaction container.

11. A kit comprising the composition of any one of claims 1 to 6 and / or adopted for carrying out the method of claim 7 or 8 and an instruction manual.
